(19) Europäisches Patentamt
European Patent Office
Office européen des brevets

(11) Publication number: 0 274 146 B1

(12) # EUROPEAN PATENT SPECIFICATION

(45) Date of publication of patent specification: **01.04.92**

(51) Int. Cl.5: **C12P 7/40**, C12P 17/00, C12P 41/00

(21) Application number: **87202349.4**

(22) Date of filing: **26.11.87**

(54) **Process for the preparation of 2-arylpropionic acids.**

(30) Priority: **01.12.86 GB 8628693**

(43) Date of publication of application:
**13.07.88 Bulletin 88/28**

(45) Publication of the grant of the patent:
**01.04.92 Bulletin 92/14**

(84) Designated Contracting States:
**AT BE CH DE ES FR GB GR IT LI LU NL SE**

(56) References cited:
**EP-A- 0 098 533**
**EP-A- 0 103 887**
**EP-A- 0 153 474**
**EP-A- 0 205 215**
**GB-A- 2 160 866**

(73) Proprietor: **GIST-BROCADES N.V.**
**Wateringseweg 1**
**NL-2611 XT Delft(NL)**

Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Bertola, Mauro Attilio**
**A. Van Scheltemaplein 91**
**NL-2624 PJ Delft(NL)**
Inventor: **Marx, Arthur Friedrich**
**Fl. Nightingalelaan 12**
**NL-2614 GM Delft(NL)**
Inventor: **Koger, Hein Simon**
**G. Vermootenstraat 32**
**NL-2064 XR Spaardam(NL)**
Inventor: **Phillips, Gareth Thomas**
**5, The Finches**
**Sittingbourne Kent(GB)**

(74) Representative: **Huygens, Arthur Victor, Dr. et al**
**c/o Gist-Brocades N.V. Patent & Trademarks Department Wateringseweg 1 PO Box 1**
**NL-2600 MA Delft(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a stereospecific process for the preparation of 2-arylpropionic acids, preferably of a pharmaceutically active compound in at least 70% by weight S-configuration.

Many biologically active compounds are synthesized in the form of a mixture of stereoisomers. Such mixtures are frequently used as such in agricultural and pharmaceutical applications. Usually the desired biological activity resides predominantly in only one stereoisomer so that where the mixture contains two or more stereoisomers, the potency of the mixture is reduced. A major reason for the continued use of mixtures of stereoisomers is that the cost of separation of the stereoisomers exceeds the potential advantage of a possible increase in activity. However, it is apparent that modern pharmacologists are becoming increasingly aware of other implications of the administration of mixtures wherein one or more stereoisomers have to be regarded merely as impurities that may not have the desired therapeutic effect, but even may have other undesired physiological effects including toxicity.

More particularly it is known that the in vitro anti-inflammatory activity of naproxen as well as that of ibuprofen resides in the S-enantiomer (optically active stereoisomer) which is up to 150 times as active as its antipode (S. Adams et al, J. Pharm. Pharmac., 28 (1976) 256 and A.J. Hutt and J. Caldwell, Clinical Pharmacokinetics 9 (1984) 371).

The selective preparation of the inactive R-enantiomer of ibuprofen by microbial oxidation of 1-isobutyl-4-(1′-methyloctyl) benzene, an aromatic hydrocarbon which has an appropriate non-functional aliphatic side chain, is known from T. Sugai and K. Mori (Agric. Biol. Chem., 48 (1984) 2501).

There is still a large need for a process on an industrial scale in an economically attractive way for the preparation of these S-enantiomers. Therefore an object of the present invention is to provide such a process.

As a result of extensive research and experimentation a stereoselective process has now surprisingly been found for the preparation of 2-arylpropionic acids, preferably a pharmaceutically active compound in at least 70% by weight S-configuration of the formula

```
          CH3
         /
R1  -  CH                 (I)
         \
          COOH
```

or a pharmaceutically acceptable salt or ester thereof, wherein $R_1$ represents an optionally substituted aryl group such as a phenyl or naphthyl group, or $R_1$ represents a heterocyclic ring system, which is optionally substituted, or $R_1$ represents a heteroaromatic ring system containing in addition to carbon atoms one or more nitrogen, sulphur or oxygen atoms, this ring system being optionally substituted, which comprises subjecting a compound of the formula

```
          CH3
         /
R1  -  CH                 (II)
         \
          R2
```

wherein $R_1$ is as defined before and $R_2$ is an alkyl group containing at least 3 carbon atoms, to the action of a micro-organism or substances derived therefrom, capable of stereoselective oxidation of compound (II) into compound (I), in predominantly S-configuration, and if desired converting compound (I) into a pharmaceutically acceptable salt or ester thereof.

By the term "predominantly in S-configuration", we mean that more than 50% by weight of the resulting compound (I) has S-configuration and in practice, according to our invention a micro-organism or substances derived therefrom is used capable of stereoselective oxidation of compound (II) into compound (I) with at least 70% by weight S-configuration, preferably at least 80% by weight and particularly at least 90% by weight S-configuration.

The pharmaceutically acceptable salt or ester of compound (I) is preferably an alkali metal salt or an alkaline earth metal salt. $R_2$ is an optionally substituted alkyl group containing at least 3 carbon atoms, for

example, forming with the $CH_3$-CH- group pentane, heptane or nonane. Preferably compound (I) is naproxen, ibuprofen, suprofen, fenoprofen, ketoprofen, benoxaprofen, carprofen, cicloprofen, pirprofen, flurbiprofen, fluprofen, tetriprofen, hexaprofen, indoprofen, mexoprofen, pranoprofen, furaprofen, protizinic acid, tiaprofenic acid or brofezil.

According to a preferred embodiment of the present invention, naproxen or ibuprofen is prepared in predominantly the S-configuration and $R_2$ is a linear alkyl containing 3-11 carbon atoms.

Compound (II) can be obtained using known methods or methods devisable for a person skilled in the art. For example, 2-(6-methoxy-2-naphthyl)alkanes are synthesized starting from 2-bromo-6-methoxy-naphthalene. The latter compound is reacted with a 2-alkanone resulting in the 2-hydroxy-2-(6-methoxy-2-naphthyl)alkane. Subsequently the product is converted into 2-(6-methoxy-2-naphthyl)-alkene-2 and hydrogenated, resulting into the 2-(6-methoxy-2-naphthyl)alkane.

Not only the compound (I) enriched in S-isomer can be obtained according to the process of the invention, compound (II) enriched in the R-isomer is a product of this process as well. The latter remains when the S-isomer is converted by a micro-organism or substances derived therefrom.

As distinct from the oxidation of 2-arylpropane, with a possible stereoselective oxidation with 100% conversion in S-2-arylpropionic acids, the oxidation of compound (II) results in a conversion of maximal 50% starting with a racemic mixture of compound (II).

The microorganisms used in the process according to the invention are capable not only of stereoselectively oxidizing the compound (II), but also oxidize only in the first carbon site of $R_2$.

By the term "micro-organisms having the ability for stereoselective oxidation" is meant, for example fungi, yeast-like fungi, yeasts and bacteria and include micro-organisms belonging to the genera Exophiala, Rhinocladiella, and Graphium.

Micro-organisms capable of the oxidation of compounds (II) into compound (I), include Exophiala jeanselmei (a strain of this species is deposited as example with CBS under the number accession number of 537.76), Exophiala mansonii (a strain of this species is deposited with CBS under the accession number of 101.67), Exophiala jeanselmei var. heteromorpha (a strain of this species is deposited with CBS under the accession number of 232.33), Rhinocladiella spinifera ( a strain of this species is deposited with CBS under the accession number 269.28), Exophiala dermatitis (a strain of this species is deposited with CBS under the accession number of 207.35), and Exophiala alcalophila (a strain of this species is deposited with CBS under the accession number of 520.82).

In an embodiment of the invention the micro-organisms capable of stereoselective oxidation of compound (II) into compound (I) may be selected from a natural source by contacting the natural source with a 2-($R_1$)-alkane ($R_1$ as defined before) as the C-source in a suitable medium. Suitably 2-(6-methoxy-2-naphthyl)alkane is used for the selection of micro-organisms having the ability for the preparation of naproxen, the alkane advantageously being pentane, heptane or nonane. A suitable medium is for example a PS III-salt medium enriched with a vitamin solution and/or 0.01% of yeast extract.

A number of micro-organisms isolated from natural sources, particularly from soil samples, were selected according to the above screening procedure, and when subjected to further tests as hereinafter described, showed their ability for stereoselective oxidation of compound (II) into compound (I).

Some of the micro-organisms used for the invention can be isolated from soil samples using 2.5 g/l 2-(6-methoxy-2-naphthyl)heptane as a C-source in PS III-salt medium enriched with a vitamin solution and/or 0.01% yeast extract.

A PS III salt medium composition contains:

$KH_2PO_4$ (2.1 g/l), $(NH_4)_2HPO_4$ (1.0 g/l), $(NH_4)_2SO_4$ (0.9 g/l), KCl (0.2 g/l), $CaSO_4 . 2 H_2O$ (0.005 g/l), $MgSO_4 . 7 H_2O$ (0.2 g/l), $(NH_4)_2SO_4.FeSO_4 . 6 H_2O$ (2.5 mg/l), $ZnSO_4 . 7 H_2O$ (0.5 mg/l), $MnCl_2 . 4 H_2O$ (0.3 mg/l), $CuSO_4 . 5 H_2O$ (0.15 mg/l), $CoCl_2 . 6 H_2O$ (0.15 mg/l), $H_3BO_3$ (0.05 mg/l), $Na_2MoO_4 . 2 H_2O$ (0.055 mg/l), KI (0.1 mg/l).

The pH was adjusted to 6.8. The medium was sterilized for 20 min. at 120 °C.

The composition of the vitamin solution contains:

Biotin (0.002 mg/l), Calcium pantothenate (0.4 mg/l), Inositol (2.0 mg/l), Nicotinic acid (0.4 mg/l), Thiamin-HCl (0.4 mg/l), Pyridoxine-HCl (0.4 mg/l), p-Aminobenzoic acid (0.2 mg/l), Riboflavin (0.2 mg/l), Folic acid, (0.01 mg/l).

The pH was adjusted to 7.0 and the medium was sterilized using a membrane filter.

The newly discovered micro-organisms were purified with agar solidified medium.

Examples of these cultures were deposited with the CBS. Micro-organisms for the oxidation of compound (II) into compound (I), obtained by the above mentioned isolation and selection method, include Graphium fructicola (a strain of this species is deposited with CBS under the accession number of 256.86 on May 15, 1986), Exophiala mansonii (a strain of this species is deposited with CBS under the accession

number of 257.86 on May 15, 1986), Exophiala mansonii (a strain of this species is deposited with CBS under the accession number of 259.86 on May 15, 1986), Exophiala jeanselmei var. jeanselmei (a strain of this species is deposited with CBS under the accession number of 258.86 on May 15, 1986) and variants or mutants thereof. These novel deposited micro-organisms form a still further embodiment of the present invention.

Also micro-organisms, which have obtained the ability for stereo-selective oxidation through the introduction of foreign genetic material are embodied by said definition. This can be accomplished by transferring the cloned gene encoding a polypeptide responsible for the stereo-selective oxidation, e.g. an enzyme, from one suitable micro-organism to another micro-organism, particularly to Escherichia coli. Other micro-organisms may be belonging to the genus Pseudomonas, Mycobacterium, Streptomyces, Saccharomyces, Kluyveromyces, Bacillus, Nocardia, Rhodococcus, Escherichia and Corynebacterium. Cloned genes may be selected for their ability to encode an enzyme capable of stereo-selective oxidation of compound (II).

Alternatively they may be selected by cross-hybridization with an already selected gene encoding an enzyme for the stereo-selective epoxidation.

The micro-organisms may advantageously be immobilized for example on a polymer gel. This can be done with living cells, killed cells and/or resting cells, or with suitable enzymes derived therefrom, which may be purified to a certain extent if a higher specific activity is needed.

Therefore by the term "micro-organisms or substances derived therefrom" is meant the micro-organisms, killed, alive or resting, and extracts therefrom such as enzymes or metabolites, optionally concentrated and/or purified. For example, enzymes optionally in combination with, for example, artificial or natural co-factors, may be used. Preferably no fermentatively active cells may be used for the oxidation of compound (II). It was found that enzymes derived from living cells or killed cells may produce the S-isomer under suitable conditions. The micro-organisms or substances derived therefrom may be used several times. Even without a co-substrate (for example glucose) the micro-organisms may remain active. The enrichment in S-isomer of compound (I) may take place in suitable buffers as well as in physiological salts solution.

In practising a preferred embodiment of the process of the present invention, a micro-organism having the ability to convert compound (II) into compound (I) with at least 70% by weight S-configuration, may be cultured for about 0.5 to 10 days. Hereafter the cells may be suspended in a liquid nutrient medium, preferably a minimal liquid nutrient medium, and compound (II) is subjected to the action of the cells. Alternatively the cells may be killed, for example suspended in a lysis medium and the 4-allyloxyphenylacetate is subjected to the action of the substances derived from the cells. After this cultivation for about 1 to 10 days, the cells may be isolated from the culture medium before suspending them in the liquid nutrient medium or suspending them in a lysis medium.

To grow the micro-organisms used for the selective oxidation of compound (II), ordinary culture mediums containing an assimilable carbon source (for example glucose, lactate, sucrose, etc.), a nitrogen source (for example ammonium sulphate, ammonium nitrate, ammonium chloride, etc.), with an agent for an organic nutrient source (for example yeast extract, malt extract peptone, meat extract, etc.) and an inorganic nutrient source (for example phosphate, magnesium, potassium, zinc, iron and other metals in trace amounts) may be used. A preferred culture medium is a Czapek-Dox, a BHI or a YEPD medium optionally enriched with one or more ingredients.

A temperature of 0 to 45 °C and a pH of 3.5 to 8 is normally maintained during the growth of the micro-organisms. Preferably the micro-organisms are grown at a temperature of 20 to 37 °C and at a pH of 4 to 7.

The aerobic conditions required during the growth of the micro-organisms can be provided according to any of the well-established procedures, provided that the supply of oxygen is sufficient to meet the metabolic requirement of the micro-organisms. This is most conveniently achieved by supplying oxygen, preferably in the form of air. During the conversion of compound (II) into compound (I) the micro-organisms might be in a growing stage using an abovementioned ordinary culture medium. The micro-organisms may be supplemented with a co-substrate.

During the conversion of compound (II) into compound (I), the micro-organisms can be held in a growing stage or in a substantially non-growing stage using a minimal culture medium. As minimal culture medium, an ordinary culture medium may be used containing an assimilable carbon source when required (for example glucose, lactate, sucrose, etc.), a nitrogen source when required (for example ammonium sulphate, ammonium nitrate, ammonium chloride, etc.), with an agent for an organic nutrient source when required (for example yeast extract, malt extract, peptone, meat extract, etc.) and an inorganic nutrient source when required (for example phosphate, magnesium, potassium, zinc, iron and other metals in trace amounts). The micro-organisms can be kept in the non-growing stage for example by exclusion of the

assimilable carbon source or by exclusion of the nitrogen source. A temperature of 0 to 45 °C and a pH of 3.5 to 8 is normally maintained during this stage. Preferably the micro-organisms are kept at a temperature of 20 to 37°C and a pH of 4 to 7. The aerobic conditions required during this stage can be provided according to the abovementioned procedures, provided that the supply of oxygen is sufficient to meet the metabolic requirement of the micro-organisms but also to convert compound (II) into compound (I). The compound (I) produced by the micro-organisms as mentioned above, can be recovered and purified according to any of the procedures known per se for such products. Advantageously the substrate to be transformed (compound II) is added to the growing or grown culture and incubated further for 5 hours to 10 days.
The invention will be illustrated by the following examples, however without restricting the scope of the invention.

Example I

The conversion of 2-(6-methoxy-2-naphthyl)-heptane into S-2-(6-methoxy-2-naphthyl) propanoic acid (S-naproxen) using fungi.

The fungal organisms were kept on agar slants or deep frozen in 50% glycerol. For the experiments they were either inoculated directly into the assay-medium or precultured in YEPD-medium at 30°C. In the case of a preculture, the organisms were grown until full growth was established after which 2-10% of the culture was inoculated into fresh assay-medium.

YEPD-medium contains: Bactopepton 20 g/l, Yeast extract 10 g/l and glucose 20 g/l. The pH was adjusted to 7.0. The medium was sterilized for 30 minutes at 110°C.

For each assay 100 ml YEPD-medium kept in a 500 ml flask was used. All assays were performed at least in duplicate. For yeast-like fungi baffle flasks were used all the time. Filamentous fungi were grown in normal flasks and transferred to baffle flasks to the time of addition of 2-(6-methoxy-2-naphthyl)-heptane.

The organisms were allowed to grow for 48 hours. Thereafter 40 µl of 2-(6-methoxy-2-naphthyl)-heptane were added to the cultures. The cultures were incubated again for 1 or 3 days at 30°C and then extracted with 40 ml dichloromethane after acidification to pH 2.0 with $H_3PO_4$ and the addition of a small amount of ammonium sulphate.

The extracts were analysed by HPLC, using a silica gel chrompack column (CP-Sper-Si) eluted with ethyl acetate/isooctane (1:7) acidified with 3.5 ml formic acid per litre.

Extracts containing compounds with the same retention time as naproxen were used for a derivatisation procedure. Naproxen present was converted into a naphthalene methylamide derivative in order to determine its enantiomeric purity.

Derivatisation procedure and separation of the enantiomers

The dichloromethane of 8 to 12 ml extract sample was evaporated under a constant stream of $N_2$ at 60°C. The dried sample was taken up in 2 ml dichloromethane and allowed to react for 10 minutes at 60°C with 200 µl of a solution of 2-bromo-1-methylpyridine iodide dissolved in dimethylformamide (50 mg/ml) and 200 µl of a solution of 1-naphthalene-methylamine dissolved in $CH_2Cl_2$ (100 mg/ml).

The reaction mixture was dried under $N_2$ at 60°C. The residue was then first dissolved in 2 ml isooctane/$CH_2Cl_2$ (2:1 v/v) and extracted after the addition of 2 ml 1 N HCl. The organic layer was then analysed by HPLC using a chiral DNBPG column eluted with isooctane/chloroform/methanol (90/7/3 v/v).

The results are presented in Table I.

Table I

| Microorganism | Timepoint after addition of substrate (days) | Naproxen* formed (mg/l) | %S | %R |
|---|---|---|---|---|
| Exophiala mansonii (CBS 257.86) | 1 | 4.5 | 74 | 26 |
| | 3 | 5.7 | 78 | 22 |
| Exophiala mansonii (CBS 259.86) | 3 | 9.5 | 73 | 27 |
| Exophiala jeanselmei var. jeanselmei (CBS 258.86) | 1 | 8.4** | 83** | 17** |
| | 3 | 18 | 99 | 1 |
| Graphium fructicola (CBS 256.86) | 3 | 6.8 | 86 | 14 |

Table I (continued)

| Microorganism | Timepoint after addition of substrate (days) | Naproxen* formed (mg/l) | %S | %R |
|---|---|---|---|---|
| Exophiala jeanselmei (CBS 537.76) | 1 | 10 | 90 | 10 |
| | 3 | 16 | 98 | 2 |
| Exophiala mansonii (CBS 101.67) | 1 | 11 | 86 | 14 |
| | 3 | 17 | 82 | 18 |
| Exophiala jeanselmei var. heteromorpha (CBS 232.33) | 3 | 2.0 | 90 | 10 |
| Rhinocladiella spinifera (CBS 269.28) | 3 | 0.8 | 100 | -- |

*The value indicated is the amount of naproxen found after derivatisation. Each quantity represents the mean of two values obtained. Only the data marked with ** were obtained from a single experiment.

Example II

The conversion of 2-(6-methoxy-2-naphthyl)-pentane into S-2-(6-methoxy-2-naphthyl)propanoic acid (S-naproxen) using fungi.

All tests were performed as described in Example I, but instead of the 2-(6-methoxy-2-naphthyl)-heptane addition, now 40 µl of 2-(6-methoxy-2-naphthyl)-pentane were added to the cultures.

Results are presented in Table II

Table II

| Microorganism | Timepoint after addition of substrate (days) | Naproxen* formed (mg/l) | %S | %R |
|---|---|---|---|---|
| Exophiala mansonii (CBS 257.86) | 3 | 11 | 75 | 25 |
| Exophiala jeanselmei var. jeanselmei (CBS 258.86) | 3 | 4.1 | 100 | - |
| Graphium fructicola (CBS 256.86) | 3 | 6.2 | 85 | 15 |

Table II (continued)

| Microorganism | Timepoint after addition of substrate (days) | Naproxen* formed (mg/l) | %S | %R |
|---|---|---|---|---|
| Exophiala dermatites (CBS 207.35) | 3 | 6.1 | 71 | 29 |
| Exophiala jeanselmei (CBS 537.76) | 3 | 21 | 98 | 2 |
| Exophiala alcalophila (CBS 520.82) | 3 | 3.1 | 79 | 21 |
| Rhinocladiella spinifera (CBS 269.28) | 3 | 0.6 | 100 | -- |

*The value indicated is the amount of naproxen found after derivatisation. Each quantity represents the mean of two values obtained.

Example III

The conversion of 2-(6-methoxy-2-naphthyl)-nonane into S-2-(6-methoxy-2-naphthyl)propanoic acid (S-naproxen) using fungi.

All tests were performed as described in Example I, but instead of the 2-(6-methoxy-2-naphthyl)-heptane addition, now 40 µl of 2-(6-methoxy-2-naphthyl)-nonane were added to the cultures.

Results are presented in Table III

Table III

| Microorganism | Timepoint after addition of substrate (days) | Naproxen* formed (mg/l) | %S | %R |
|---|---|---|---|---|
| Exophiala mansonii (CBS 257.86) | 3 | 27.6 | 80 | 20 |
| Exophiala mansonii (CBS 259.86) Exophiala jeanselmei | 3 | 22.5 | 70 | 30 |
| var. jeanselmei (CBS 258.86) | 3 | 10.8 | 100 | - |
| Graphium fructicola (CBS 256.86) | 3 | 9.3 | 73 | 27 |
| Exophiala jeanselmei (CBS 537.76) | 3 | 25** | 93** | 7** |
| Exophiala alcalophila (CBS 520.82) | 3 | 2.1** | 86** | 14** |
| Exophiala mansonii (CBS 101.67) | 3 | 2.5 | 81 | 19 |
| Rhinocladiella spinifera (CBS 269.28) | 3 | 1.0 | 100 | -- |

*The value indicated is the amount of naproxen found after derivatisation. Each quantity represents the mean of two values obtained.
Only the data marked with ** were obtained from a single experiment.

Example IV:

Isolation and identification of S-2-(6-methoxy-2-naphthyl) propanoic acid (S-naproxen) formed by the fungus Exophiala jeanselmei var. jeanselmei (CBS 258.86).

A total of 500 ml Exophiala jeanselmei var. jeanselmei (CBS 258.68) culture grown in YEPD medium in which 2-(6-methoxy-2-naphthyl) heptane was transformed into naproxen as described in Example I, was used for an isolation procedure and identification of the naproxen formed.

The culture broth was acidified and extracted several times with $CH_2Cl_2$. After evaporation of the $CH_2Cl_2$, 0.16 g residue was left over. The residue was dissolved in 10 ml diethyl ether and extracted with 3 $\times$ 5ml 1M $NaHCO_3$. The aqueous phase was acidified and reextracted with 3 $\times$ 5ml dimethylether. After washing of the organic layer with 30% NaCl, the extract was dried over $Na_2SO_4$ and after evaporation of the diethyl ether 20 mg crude residue was left over. The residue was analysed with pmr and naproxen being one of the compounds present was detected by the following characteristic signals:
$\delta$ 1.59 ppm (doublet, 3H, coupling constant 7 Hz)
$\delta$ 3.91 ppm (singlet, 3H from $OCH_3$)
$\delta$ 7.12 ppm (multiplet, 2H from naphthyl)
$\delta$ 7.42 ppm (double doublet, 1 H from naphthyl)
$\delta$ 7.70 ppm (multiplet, 3H from naphthyl)

## Claims

**1.** A stereospecific process for the preparation of a pharmaceutically active compound in at least 70% by weight S-configuration of the formula

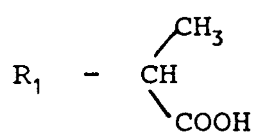

(I)

or a pharmaceutically acceptable salt or ester thereof, wherein $R_1$ represents an optionally substituted aryl group such as a phenyl or naphthyl group, or $R_1$ represents a heterocyclic ring system, which is optionally substituted, or $R_1$ represents a heteroaromatic ring system optionally containing in addition to carbon atoms one or more nitrogen, sulphur or oxygen atoms, this ring system being optionally substituted, which comprises subjecting a compound of the formula

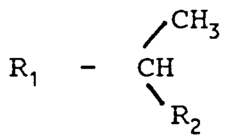 (II)

wherein $R_1$ is as defined above and $R_2$ is an alkyl group containing at least 3 carbon atoms, to the action of a micro-organism or substances derived therefrom capable of stereoselective oxidation of compound (II) into compound (I) with at least 70% by weight S-configuration, and if desired converting compound (I) into a pharmaceutically acceptable salt or ester thereof.

**2.** A process according to claim 1 wherein compound (I) is naproxen, ibuprofen, cicloprofen, suprofen, carprofen, ketoprofen, benoxaprofen, fenoprofen, pirprofen, flurbiprofen, fluprofen, tetriprofen, hexaprofen, indoprofen, mexoprofen, pranoprofen, furaprofen, protizinic acid, tiaprofenic acid or brofezil.

**3.** A process according to claim 2 wherein compound (I) is naproxen or ibuprofen.

**4.** A process according to any one of the preceding claims wherein said micro-organism or substances derived therefrom are capable of converting compound (II) into a compound (I), with at least 90% by weight S-configuration.

**5.** A process according to any one of the preceding claims, wherein said micro-organism or substances derived therefrom are isolated and selected from natural sources using a 2-($R_1$)alkane as a C-source in a suitable medium.

**6.** A process according to claim 5, wherein $R_1$ represents a 6-methoxy-2-naphthyl group and the alkane is pentane, heptane or nonane.

**7.** A process according to any one of the preceding claims, wherein said micro-organism is a fungus, a yeast, a yeast-like fungus or a bacterium.

**8.** A process according to claim 7, wherein said micro-organism belongs to the genus Graphium.

**9.** A process according to claim 7, wherein said micro-organism belongs to the genus Exophiala.

**10.** A process according to claim 7, wherein said micro-organism belongs to the genus Rhinocladiella.

**11.** A process according to any one of the preceding claims, wherein said micro-organism or substance derived therefrom is immobilized.

**12.** A process for the isolation and selection from natural sources of micro-organisms capable of stereoselective oxidation of a compound of the formula

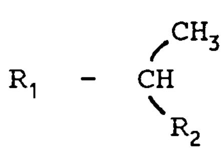 (II)

wherein $R_1$ represents an optionally substituted aryl group such a phenyl or naphthyl group, or $R_1$ represents a heterocyclic ring system, which is optionally substituted, or $R_1$ represents a heteroaromatic ring system optionally containing in addition to carbon atoms one or more nitrogen, sulphur or oxygen atoms, this ring system being optionally substituted and wherein $R_2$ is an alkyl group containing at least 3 carbon atoms, into a compound of the formula

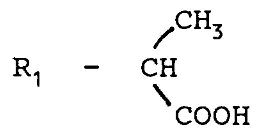

(I)

with at least 70% by weight of S-configuration, by contacting a natural source with a 2-($R_1$)alkane as C-source in a suitable medium.

**13.** A process according to claim 12 wherein $R_1$ represents a 6-methoxy-2-naphthyl group and the alkane is pentane, heptane or nonane.

**14.** The use of a micro-organism selected from the group Graphium fructicola (CBS 256.86), Exophiala mansonii (CBS 257.86), Exophiala mansonii (CBS 259.86), Exophiala jeanselmei, var. jeanselmei (CBS 258.86) and mutants or variants thereof, in a process according to claim 1.

**Revendications**

**1.** Procédé stéréospécifique de préparation d'un composé pharmaceutiquement actif ayant la configuration S pour au moins 70% en poids de la formule :

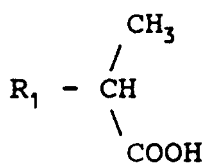

(I)

ou d'un sel ou ester pharmaceutiquement acceptable de celui-ci, où $R_1$ représente un radical aryle, comme un radical phényle ou naphtyle, facultativement substitué, ou $R_1$ représente un système hétérocyclique qui est facultativement substitué, ou bien $R_1$ représente un système hétéroaromatique contenant en plus d'atomes de carbone un ou plusieurs atomes d'azote, de soufre ou d'oxygène, ce système cyclique étant facultativement substitué, qui comprend l'exposition d'un composé de formule :

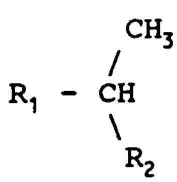

(II)

où $R_1$ est tel que défini ci-dessus et $R_2$ est un radical alcoyle contenant au moins 3 atomes de carbone, à l'action d'un micro-organisme ou de substances qui en dérivent, capables d'oxydation stéréosélective d'un composé (II) en un composé (I) ayant de façon prépondérante la configuration S, et, si la chose est souhaitée, la conversion du composé (I) en un sel ou ester pharmaceutiquement acceptable de celui-ci.

**2.** Procédé suivant la revendication 1, dans lequel le composé (I) est le naproxène; l'ibuprofène, le cycloprofène, le suprofène, le carprofène, le cétoprofène, le bénoxaprofène, le fénoprofène, le pirprofène, le flurbiprofène, le fluprofène, le tétriprofène, l'hexaprofène, l'indoprofène, le méxoprofène, le pranoprofène, le furaprofène, l'acide protizinique, l'acide tiaprofénique ou le brofézile.

**3.** Procédé suivant la revendication 2, dans lequel le composé (I) est le naprofène ou l'ibuprofène.

**4.** Procédé suivant l'une quelconque des revendications précédentes, dans lequel le micro-organisme ou les substances qui en dérivent sont capables de convertir le composé (II) en un composé (I) ayant la configuration S pour au moins 90% en poids.

**5.** Procédé suivant l'une quelconque des revendications précédentes, dans lequel le micro-organisme ou les substances qui en dérivent sont isolés et sélectionnés de sources naturelles au moyen d'un 2-($R_1$)-alcane comme source de C dans un milieu approprié.

**6.** Procédé suivant la revendication 5, dans lequel $R_1$ représente un radical 6-méthoxy-2-naphtyle et l'alcane est le pentane, l'heptane ou le nonane.

**7.** Procédé suivant l'une quelconque des revendications précédentes, dans lequel le micro-organisme est un champignon, une levure, un champignon semblable à une levure ou une bactérie.

**8.** Procédé suivant la revendication 7, dans lequel le micro-organisme appartient au genre Graphium.

**9.** Procédé suivant la revendication 7, dans lequel le micro-organisme appartient au genre Exophiala.

**10.** Procédé suivant la revendication 7, dans lequel le micro-organisme appartient au genre Rhinocladiella.

**11.** Procédé suivant l'une quelconque des revendications précédentes, dans lequel le micro-organisme ou la substance qui en dérive est immobilisé.

**12.** Procédé d'isolement et de sélection à partir de sources naturelles de micro-organismes capables d'oxydation stéréospécifique d'un composé de formule :

```
          CH3
         /
R1  -  CH                         (II)
         \
          R2
```

où $R_1$ représente un radical aryle, comme un radical phényle ou naphtyle, facultativement substitué, ou $R_1$ représente un système hétérocyclique qui est facultativement substitué, ou bien $R^1$ représente un système hétéroaromatique contenant facultativement en plus d'atomes de carbone, un ou plusieurs atomes d'azote, de soufre ou d'oxygène, ce système cyclique étant facultativement substitué, et où $R_2$ est un radical alcoyle contenant au moins 3 atomes de carbone, en un composé de formule :

```
          CH3
         /
R1  -  CH                         (I)
         \
          COOH
```

ayant la configuration S pour au moins 70% en poids, par mise en contact d'une source naturelle avec un 2-($R_1$)-alcane comme source de C dans un milieu approprié.

**13.** Procédé suivant la revendication 12, dans lequel $R_1$ représente un radical 6-méthoxy-2-naphtyle et l'alcane est le pentane, l'heptane ou le nonane.

**14.** Utilisation d'un micro-organisme choisi dans la classe formée par Graphium fructicola (CBS 256.86), Exophiala mansonii (CBS 257.86), Exophiala mansonii (CBS 259.86), Exophiala jeanselmei, var. jeanselmei (CBS 258.86) et leurs mutants ou variants, dans un procédé suivant la revendication 1.

**Patentansprüche**

**1.** Stereospezifisches Verfahren für die Herstellung von pharmazeutisch aktiven Verbindungen, die zu mindestens 70 Gew.-% S-Konfiguration aufweisen, der Formel:

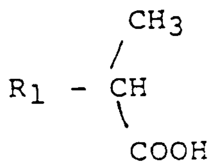

(I)

oder einem pharmazeutisch unbedenklichen Salz oder Ester davon, worin $R_1$ eine gegebenenfalls substituierte Arylgruppe, z.B. eine Phenyl- oder Naphthylgruppe, darstellt oder $R_1$ ein heterocyclisches Ringsystem, welches gegebenenfalls substituiert ist, darstellt oder $R_1$ ein heteroaromatisches Ringsystem, welches gegebenenfalls zusätzlich zu Kohlenstoffatomen ein oder mehr Stickstoff-, Schwefel- oder Sauerstoffatome aufweist, wobei dieses Ringsystem gegebenenfalls substituiert ist, darstellt, dadurch gekennzeichnet, dass man eine Verbindung der Formel:

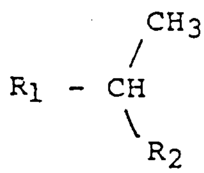

(II)

worin $R_1$ die obige Bedeutung hat und $R_2$ eine mindestens 3 Kohlenstoffatome enthaltende Alkylgruppe darstellt, der Einwirkung eines Mikroorganismus oder von sich davon ableitenden Substanzen, welche zu einer stereoselektiven Oxidation der Verbindung der Formel II zur Verbindung der Formel I, die zu mindestens 70 Gew.-% S-Konfiguration aufweist, befähigt sind, unterwirft und gewünschtenfalls die Verbindung der Formel I in ein pharmazeutisch unbedenkliches Salz oder einen pharmazeutisch unbedenklichen Ester davon überführt.

**2.** Verfahren nach Anspruch 1, dadurch gekennzeichnet, dass die Verbindung der Formel I Naproxen, Ibuprofen, Cicloprofen, Suprofen, Carprofen, Ketoprofen, Benoxaprofen, Fenoprofen, Pirprofen, Flurbiprofen, Fluprofen, Tetriprofen, Hexaprofen, Indoprofen, Mexoprofen, Pranoprofen, Furaprofen, Protizinic acid, Tiaprofenic acid oder Brofezil ist.

**3.** Verfahren nach Anspruch 2, dadurch gekennzeichnet, dass die Verbindung der Formel I Naproxen oder Ibuprofen ist.

**4.** Verfahren nach einem der vorangehenden Ansprüche, dadurch gekennzeichnet, dass die besagten Mikroorganismen oder sich davon ableitenden Substanzen eine Verbindung der Formel II in eine Verbindung der Formel I überzuführen vermögen, die zu mindestens 90 Gew.-% S-Konfiguration aufweist.

**5.** Verfahren nach einem der vorangehenden Ansprüche, dadurch gekennzeichnet, dass die Mikroorganismen oder sich davon ableitenden Substanzen aus natürlichen Quellen isoliert und unter Verwendung eines 2-($R_1$)-Alkans als C-Quelle in einem geeigneten Medium selektioniert werden.

**6.** Verfahren nach Anspruch 5, dadurch gekennzeichnet, dass $R_1$ eine 6-Methoxy-2-naphthylgruppe darstellt und das Alkan pentan, Heptan oder Nonan ist.

**7.** Verfahren nach einem der vorangehenden Ansprüche, dadurch gekennzeichnet, dass der Mikroorganismus ein Pilz, eine Hefe, ein hefeähnlicher Pilz oder ein Bakterium ist.

**8.** Verfahren nach Anspruch 7, dadurch gekennzeichnet, dass der Mikroorganismus der Gattung Graphium angehört.

**9.** Verfahren nach Anspruch 7, dadurch gekennzeichnet, dass der Mikroorganismus der Gattung Exophiala

angehört.

**10.** Verfahren nach Anspruch 7, dadurch gekennzeichnet, dass der Mikroorganismus der Gattung Rhinocladiella angehört.

**11.** Verfahren nach einem der vorangehenden Ansprüche, dadurch gekennzeichnet, dass der besagte Mikroorganismus oder die sich davon ableitende Substanz immobilisiert ist.

**12.** Verfahren zur Isolierung und Selektion aus natürlichen Quellen von Mikroorganismen, die zur stereoselektiven Oxidation einer Verbindung der Formel

```
          CH3
         /
R1  -  CH                (II)
         \
          R2
```

worin $R_1$ eine gegebenenfalls substituierte Arylgruppe, z.B. eine Phenyl- oder Naphthylgruppe, darstellt oder $R_1$ ein heterocyclisches Ringsystem, welches gegebenenfalls substituiert ist, darstellt oder $R_1$ ein heteroaromatisches Ringsystem, welches gegebenenfalls zusätzlich zu Kohlenstoffatomen ein oder mehr Stickstoff-, Schwefel- oder Sauerstoffatome enthält, wobei dieses Ringsystem gegebenenfalls substituiert ist, darstellt und worin $R_2$ eine mindestens 3 Kohlenstoffatome enthaltende Alkylgruppe darstellt, zu einer Verbindung der Formel

```
          CH3
         /
R1  -  CH                (I)
        \
         COOH
```

die zu mindestens 70 Gew.-% S-Konfiguration aufweist, befähigt sind, durch Kontaktieren einer natürlichen Quelle mit einem 2-($R_1$)-Alkan als C-Quelle in einem geeigneten Medium.

**13.** Verfahren nach Anspruch 12, dadurch gekennzeichnet, dass $R_1$ eine 6-Methoxy-2-naphthylgruppe darstellt und das Alkan Pentan, Heptan oder Nonan ist.

**14.** Verwendung eines Mikroorganismus, der aus der Gruppe gewählt ist, die aus Graphium fructicola (CBS 256.86), Exophiala mansonii (CBS 257.86), Exophiala mansonii (CBS 259.86), Exophiala jeanselmei, var. jeanselmei (CBS 258.86) und Mutanten oder Varianten davon besteht, in einem Verfahren gemäss Anspruch 1.